# FASCICULE DE BREVET EUROPEEN

(11) **EP 2 406 757 B1**
(45) Date de publication et mention de la délivrance du brevet: **31.10.2012**
(21) Numéro de dépôt: 09805795.3
(22) Date de dépôt: 28.12.2009
(51) Int. Cl.: G06M 1/16, G06M 1/24

(54) **COMPTEUR POUR DISPOSITIF DE DISTRIBUTION DE PRODUIT FLUIDE OU PULVERULENT**
ZÄHLER FÜR EINE VORRICHTUNG ZUR ABGABE EINES FLUID- ODER PULVERPRODUKTS
METER FOR A DEVICE FOR DISPENSING A FLUID OR POWDER PRODUCT

(30) Priorité: 30.12.2008 FR 0859140
(43) Date de publication de la demande: 18.01.2012
(73) Titulaire: Aptar France SAS, 27110 Le Neubourg (FR)
(72) Inventeur: LAUT, Antoine, F-27150 Etrepagny (FR)
(74) Mandataire: CAPRI
(86) Numéro de dépôt international: PCT/FR2009/052708
(87) Numéro de publication internationale: WO 2010/076531

(56) Documents cités:
- WO-A-2005/114563
- WO-A-2007/077450
- WO-A-2007/104964

## Description

La présente invention concerne un compteur, et plus particulièrement un compteur de doses pour indiquer à l'utilisateur le nombre de doses distribuées ou restant à distribuer à partir d'un dispositif de distribution de produit fluide ou pulvérulent.

L'utilisation de compteurs ou d'indicateurs est bien connue dans le domaine des distributeurs de produit fluide, notamment dans le domaine pharmaceutique. Ces compteurs ou indicateurs sont notamment utilisés avec des dispositifs de distribution du type MDI (Metered Dose Inhalers), dans lesquels un réservoir contenant du produit fluide et un gaz propulseur est monté déplaçable dans un corps, le déplacement dudit réservoir provoquant l'actionnement d'une valve doseuse montée sur ledit réservoir pour distribuer une dose de produit. Une première famille de compteurs prévoit de fixer le compteur sur le fond du réservoir faisant saillie hors du corps, et sur lequel l'utilisateur appuie pour distribuer une dose. Ce type de compteur présente toutefois l'inconvénient d'interférer avec l'actionnement du dispositif de distribution, l'utilisateur devant appuyer sur le compteur pour actionner le dispositif. En cas d'actionnement mal maîtrisé ou partiel, il peut alors se poser des problèmes de sur- et/ou sous-comptage et/ou de distribution incomplète ou défaillante. Une seconde famille de compteurs comprend des compteurs disposés à l'intérieur du corps en étant fixés soit au corps, soit au réservoir mobile dans ledit corps. Ce type de compteur présente notamment l'inconvénient d'un montage complexe et nécessite des modifications substantielles des différentes parties constitutives du dispositif de distribution. Le problème de l'assemblage se pose notamment lorsque cet assemblage est réalisé chez le fabricant du produit pharmaceutique et non chez le fabricant du dispositif de distribution, obligeant le fabricant du produit à installer des machines d'assemblages complexes dans son usine. Une troisième famille de compteurs prévoit de disposer le compteur latéralement à l'extérieur du corps, une projection dudit compteur passant à travers une ouverture du corps pour coopérer avec le réservoir ou une partie solidaire de celui-ci. Ce type de compteurs nécessite généralement aussi une modification substantielle du corps afin de recevoir le compteur. Par ailleurs, la présence d'un compteur sur la face latérale externe du corps modifie sensiblement l'aspect extérieur du dispositif, en particulier en raison de l'épaisseur dudit compteur, ce qui peut aussi avoir un effet négatif sur la maniabilité du dispositif. Par ailleurs, plusieurs contraintes reposent sur des compteurs utilisés avec des dispositifs de distribution de produits, notamment pharmaceutiques. Ainsi, pour éviter tout risque de sous-comptage, il est généralement exigé que l'actionnement du compteur soit réalisé au tout début de la course d'actionnement de la valve ou de la pompe, pour éviter qu'un actionnement partiel ne distribue une dose partielle ou complète, mais qui ne serait pas comptée par le compteur. Un problème qui se pose dans ce cas est que cette course d'actionnement est généralement très courte et que les tolérances du dispositif ont tendance à encore d'avantage réduire la distance disponible de manière effective pour réaliser cet actionnement. Ceci nécessite généralement l'utilisation d'un mécanisme complexe pour fournir un comptage fonctionnel et sûr. De manière générale, l'assemblage des compteurs, notamment ceux comportant plusieurs éléments rotatifs imbriqués les uns dans les autres, s'avère complexe et donc non seulement coûteuse mais aussi sources de dysfonctionnements. Les documents WO 2007/104964, WO 2007/077450 et WO 2005/114563 décrivent des dispositifs de l'art antérieur.

La présente invention a pour but de fournir un compteur, plus particulièrement un compteur de doses pour dispositif de distribution de produit fluide ou pulvérulent, qui ne reproduit pas les inconvénients susmentionnés.

La présente invention a notamment pour but de fournir un tel compteur qui présente une épaisseur minimale.

La présente invention a aussi pour but de fournir un tel compteur qui puisse être pré-assemblé avant livraison au fabricant du produit pharmaceutique, celui-ci n'ayant plus alors qu'à réaliser une simple étape de montage du compteur sur le corps du dispositif de distribution, sans assemblage complexe des parties constitutives dudit compteur.

La présente invention a aussi pour but de fournir un tel compteur qui garantit un actionnement du compteur indépendamment de la longueur de la course d'actionnement de la pompe ou de la valve utilisée dans le dispositif.

La présente invention a encore pour but de fournir un tel compteur qui soit plus simple et donc moins coûteux à fabriquer et à assembler, et plus fiable de fonctionnement.

La présente invention a donc pour objet un compteur de doses selon la revendication 1.

Selon la revendication 2, ladite première denture est radialement à l'intérieur de la seconde denture, les dents de la première denture étant orientées axialement et les dents de la seconde denture étant orientées radicalement

Selon la revendication 3, ledit premier élément de comptage est sensiblement en forme de disque pourvu d'une ouverture centrale traversante adaptée à s'emmancher autour de son axe de rotation, la face supérieure dudit disque comportant une première partie de bord périphérique radialement externe recevant des indices de comptage, tel que une ou plusieurs séries de chiffres de 0 à 9, notamment trois séries de chiffres de 0 à 9, répartis sur ladite périphérie, la face inférieure dudit disque comportant lesdites première et seconde dentures périphériques.

Selon la revendication 4, la face supérieure dudit premier élément de comptage comporte une partie centrale entourant l'ouverture centrale, prolongée radialement à l'extérieur par une partie intermédiaire axialement surélevée par rapport à ladite partie centrale, ladite partie intermédiaire étant prolongée radialement à l'extérieur par ladite première partie de bord périphérique axialement surélevée par rapport à ladite partie intermédiaire.

Selon la revendication 5, ledit second élément de comptage est sensiblement en forme de disque pourvu d'un orifice central, notamment borgne, adapté à s'emmancher autour de son axe de rotation, la face supérieure dudit disque comportant une seconde partie de bord périphérique radialement externe recevant des indices de comptage, tel que les chiffres de 00 à 20, répartis sur ladite périphérie, la face supérieure dudit disque comportant également ladite troisième denture périphérique, disposée radialement à l'intérieur de ladite seconde partie de bord périphérique externe, ladite troisième denture périphérique étant axialement surélevée par rapport à ladite seconde partie de bord périphérique externe.

Selon la revendication 6, après assemblage des premier et second éléments de comptage autour de leur axe de rotation commun, ladite seconde partie de bord périphérique externe dudit second élément de comptage est disposée radialement à l'intérieur et sensiblement en contact de ladite première partie de bord périphérique externe dudit premier élément de comptage, les surfaces supérieures desdites première et secondes parties de bord périphérique étant sensiblement alignées pour former une zone d'affichage.

Selon la revendication 7, ledit élément intermédiaire comporte une quatrième denture périphérique coopérant à chaque actionnement de l'organe d'actionnement avec une seconde denture périphérique dudit premier élément de comptage.

Selon la revendication 8, ledit élément intermédiaire comporte au moins une projection radiale coopérant tous les dix actionnements de l'organe d'actionnement avec une troisième denture périphérique du second élément de comptage.

Selon la revendication 9, ladite au moins une projection radiale est formée sur une partie de tige radiale dudit élément intermédiaire.

Selon la revendication 10, ledit élément intermédiaire comporte un manchon creux axial central, notamment borgne, définissant un orifice central adapté à s'emmancher autour de son axe de rotation, ladite quatrième denture et ladite partie de tige radiale étant décalées l'une de l'autre le long dudit manchon en définissant entre elles un espace destiné à recevoir une première partie de bord périphérique externe du premier élément de comptage et une seconde partie de bord périphérique externe du second élément de comptage.

Selon la revendication 11, ladite partie de tige radiale comporte deux projections radialement externes diamétralement opposées reliées entre elles par une zone de tige cintrée dont les bords latéraux sont de forme sensiblement en arc de cercle inversés, de sorte que dans une orientation appropriée, les premier et second éléments de comptage peuvent être assemblés sur leur axe de rotation commun après assemblage de l'élément intermédiaire sur son propre axe de rotation.

Selon la revendication 12, le compteur comporte un corps de base et un couvercle, ledit corps de base incorporant les deux axes de rotation et une ouverture et ledit couvercle incorporant une fenêtre de visualisation.

Selon la revendication 13, ledit compteur peut être pré-assemblé pour former une unité de compteur, ladite unité de compteur comportant des moyens de fixation un corps d'un dispositif de distribution de produit fluide.

Selon la revendication 14, ledit organe d'actionnement est assemblé dans ledit corps de base, ledit organe d'actionnement comportant un élément d'actionnement déplaçable en translation dans ladite ouverture et deux pattes flexibles déplaçables en translation, une première patte flexible coopérant avec ladite première denture du premier élément de comptage pour faire tourner ledit premier élément de comptage dans une direction de comptage chaque fois que l'élément d'actionnement est déplacé d'une position de repos vers une position d'actionnement, et une seconde patte flexible coopérant avec ladite première denture dudit premier élément de comptage pour faire tourner ledit premier élément de comptage dans la même direction de comptage lorsque l'élément d'actionnement revient de sa position d'actionnement vers sa position de repos.

Selon la revendication 15, la première patte flexible pousse une première dent respective de la première denture du premier élément de comptage à chaque actionnement, et la seconde patte flexible tire sur une seconde dent respective de la première denture dudit premier élément de comptage à chaque actiomement.

Selon la revendication 16, les première et seconde dents de la première denture sont sensiblement diamétralement opposées sur la première denture.

Selon la revendication 17, l'organe d'actionnement comporte une ouverture allongée assemblée autour d'une projection dudit corps de base, la coopération entre ladite projection et les bords de ladite ouverture allongée lors de l'actionnement de l'organe d'actionnement définissant les limites du déplacement axial desdites pattes flexibles.

Selon la revendication 18, l'organe d'actionnement comporte une partie élastiquement déformable supportant l'élément d'actionnement de sorte que ledit élément d'actionnement est déplaçable en translation sur une plus grande distance que les pattes flexibles.

Selon la revendication 19, ledit corps de base comporte deux épaulements coopérant avec deux moyens élastiques de l'organe d'actionnement, lesdits moyens élastiques formant ressort de rappel pour l'organe d'actionnement.

Selon la revendication 20, ledit couvercle comporte une patte flexible coopérant avec une troisième denture dudit second élément de comptage pour empêcher ledit second élément de comptage de tourner pendant les actionnements de l'organe d'actionnement dans lesquels l'élément intermédiaire ne coopère pas avec ledit second élément de comptage, ladite patte flexible se déformant élastiquement pour permettre une rotation dudit second élément de comptage lorsque ledit élément intermédiaire coopère avec ledit second élément de comptage tous les dix actionnements dudit organe d'actionnement.

Selon la revendication 21, un dispositif de distribution de produit fluide ou pulvérulent comporte un réservoir, un organe de distribution, tel qu'une valve doseuse, monté sur ledit réservoir, et un corps incorporant un orifice de distribution, ledit réservoir étant déplaçable dans ledit corps pour distribuer du produit fluide ou pulvérulent, ledit dispositif de distribution comportant un compteur tel que décrit dans les revendications 1-20.

Selon la revendication 22, ledit compteur est fixé latéralement au corps, l'actionnement dudit dispositif selon la revendication 21, étant réalisé par une pression manuelle axiale de l'utilisateur sur le réservoir et l'actionnement dudit compteur étant réalisé par ledit déplacement axial dudit réservoir qui coopère avec ledit élément d'actionnement de l'organe, d'actionnement.

Selon la revendication 23, un procédé d'assemblage d'un compteur selon les revendications 1-20, comporte les étapes de fournir un corps de base pourvu de deux projections parallèles et d'une ouverture, d'assembler un organe d'actionnement dans ledit corps de base, ledit organe d'actionnement comportant un élément d'actionnement s'étendant hors dudit corps de base à travers ladite ouverture du corps de base, d'assembler un élément intermédiaire rotatif sur une première desdites deux projections dudit corps de base, d'assembler un premier élément de comptage rotatif sur une seconde desdites deux projections dudit corps de base, d'assembler un second élément de comptage rotatif sur ladite seconde projection dudit corps de base, et d'assembler un couvercle sur ledit corps de base, pour former une unité de compteur pré-assemblée.

Ces caractéristiques et avantages et d'autres de la présente invention apparaîtront plus clairement au cours de la description détaillée suivante, faite en référence aux dessins joints , donnés à titre d'exemples non limitatifs, et sur lesquels :
- la figure 1 est une vue éclatée en perspective d'un dispositif de distribution comportant sur sa face latérale avant compteur selon un mode de réalisation particulier de la présente invention ;
- les figures 2a et 2b sont deux vues schématiques en perspectives du compteur pré-assemblé de la figure 1, respectivement vue de devant et de derrière ;
- la figure 3 est une vue schématique en perspective de l'organe d'actionnement selon un mode de réalisation avantageux de l'invention ;
- la figure 4 est une vue schématique en perspective partiellement transparente des deux éléments de comptage rotatifs et de l'élément intermédiaire rotatif, selon un mode de réalisation avantageux de l'invention, en position d'assemblage de l'élément intermédiaire ;
- la figure 5 est une vue schématique découpée en perspective du compteur selon un mode de réalisation avantageux,
- la figure 6 est une vue schématique en coupe transversale du compteur de la figure 5,
- les figures 7a et 7b représentent des vues schématiques respectivement de devant et de derrière du premier élément de comptage rotatif, selon une variante de réalisation avantageuse,
- les figures 8a et 8b représentent des vues schématiques respectivement de devant et de derrière du second élément de comptage rotatif, selon une variante de réalisation avantageuse, et
- les figures 9a et 9b représentent des vues schématiques respectivement de devant et de derrière de l'élément intermédiaire rotatif, selon une variante de réalisation avantageuse.

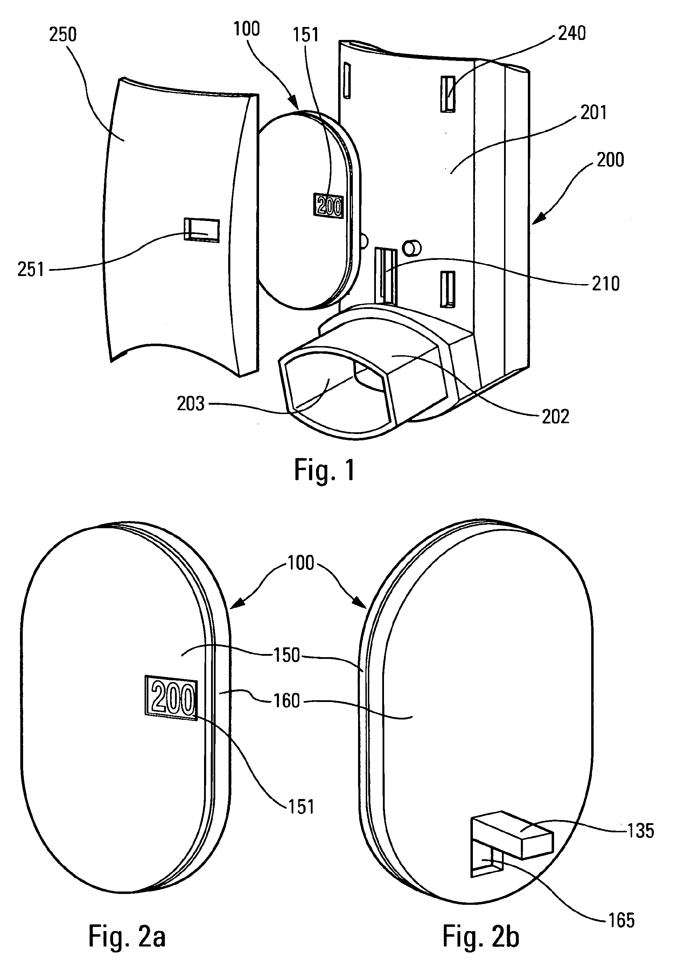

En référence à la figure 1, il est représenté un dispositif de distribution du type MDI 200, comportant sur sa face latérale avant un compteur de doses 100 qui correspond à un mode de réalisation particulier de la présente invention. Le dispositif comporte un corps 201 pourvu d'un embout buccal 202 définissant un orifice de distribution 203. Un réservoir (non représenté) est assemblé dans ledit corps 201, ledit réservoir comportant une valve doseuse monté sur son ouverture. Cette valve doseuse coopère à l'intérieur dudit corps 201 avec un canal d'expulsion menant dans l'embout buccal 202. Lorsque l'utilisateur appuie sur le fond du réservoir, il fait coulisser celui-ci axialement à l'intérieur du corps 201, provoquant l'actionnement de la valve et l'expulsion d'une dose de produit fluide. Le fonctionnement d'un tel dispositif de type MDI est bien connu de l'homme du métier, et il ne sera donc pas plus amplement décrit ici. Le compteur 100 peut être intercalé entre la face latérale avant dudit corps 201 et un organe de recouvrement 250 comportant une fenêtre 251, et qui peut servir à fixer le compteur 100 sur le corps. Bien entendu, cette mise en oeuvre n'est qu'un exemple de réalisation, et par exemple, le compteur pourrait directement être fixé sur le corps 201, indépendamment de la présence d'un organe de recouvrement. Le compteur comporte un élément d'actionnement 135 (non visible sur la figure 1, mais clairement représentée sur la figure 2b) qui se projette hors dudit compteur, et qui est adapté à pénétrer à l'intérieur du corps 201 à travers une ouverture 210 prévue à cet effet, pour coopérer avec le réservoir ou une partie solidaire de celui-ci. De cette manière, à chaque actionnement du dispositif de distribution, le déplacement axial du réservoir dans le corps 201 provoque le déplacement axial de l'élément d'actionnement 135.

Le compteur selon l'invention comporte deux éléments de comptage rotatifs, à savoir un premier élément de comptage rotatif 110 et un second élément de comptage rotatif 120, un organe d'actionnement 130 et un élément intermédiaire rotatif 140. L'organe d'actionnement 130, qui comporte l'élément d'actionnement 135, est prévu pour transformer un déplacement axial d'une partie du dispositif de distribution 200, en général le réservoir, en un déplacement rotatif du premier élément de comptage 110.

Dans une variante de réalisation préférée, représentée sur la figure 1, le compteur est disposé latéralement par rapport au corps 201 du dispositif de distribution 200, et l'organe d'actionnement 130 transforme alors un déplacement axial du réservoir en un déplacement rotatif du premier élément de comptage 110. Dans ce cas, les trois éléments rotatifs 110, 120, 140 du compteur tournent autour d'axes de rotation 161, 162 sensiblement perpendiculaires à ce déplacement axial. Avantageusement, le cycle d'actionnement du compteur peut démarrer au tout début de la course du réservoir, de sorte que le compteur est actionné avant que le produit ne soit distribué.

Le premier élément de comptage rotatif 110 forme la roue des unités et le second élément de comptage rotatif 120 forme la roue des dizaines, lesdits premier et second éléments de comptage coopérant pour définir ensemble et afficher dans une fenêtre de visualisation 151 le nombre de doses de produit fluide distribué ou restant à distribuer dudit réservoir. De préférence, ce nombre est formé par une zone d'affichage dans laquelle le nombre est affiché horizontalement lorsque le dispositif de distribution 200 est dans sa position d'utilisation normale, représentée sur la figure 1, dans laquelle le corps 201 est sensiblement vertical avec l'embout buccal 202 disposé en bas. Ledit premier élément de comptage 110 coopère avec l'organe d'actionnement 130 qui est adapté à faire tourner ledit premier élément de comptage 110 à chaque actionnement dudit organe d'actionnement. L'élément intermédiaire rotatif 140 est adapté à faire tourner ledit second élément de comptage 120 tous les dix actionnements dudit organe d'actionnement 130, et donc toutes les dix rotations dudit premier élément de comptage 110. Selon l'invention, lesdits premier et second éléments de comptage 110, 120 tournent autour d'un même premier axe de rotation 161, et ledit élément intermédiaire 140 tourne autour d'un second axe de rotation 162, décalé et parallèle audit premier axe de rotation 161.

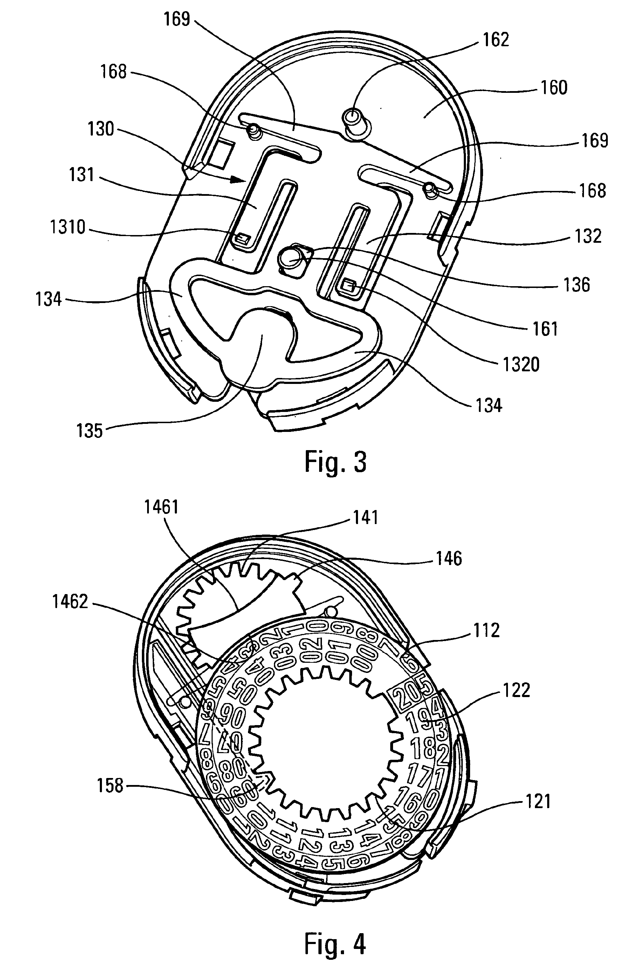

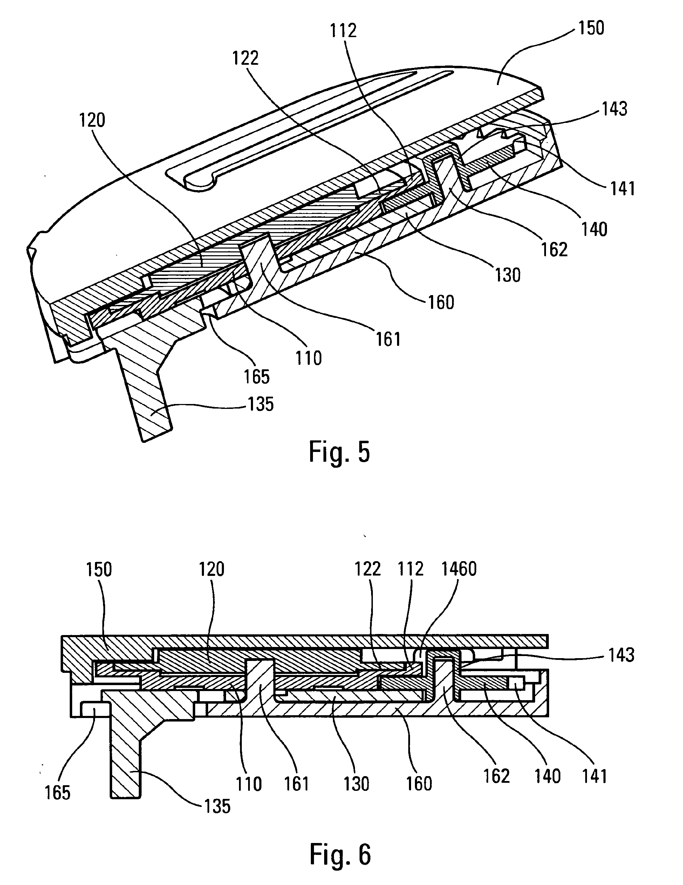

Comme représenté sur les figures 3, 5 et 6, le compteur comporte de préférence un corps de base 160 et un couvercle 150. Ledit corps de base forme les deux axes de rotation 161 et 162 et comporte une ouverture 165 destinée à laisser passer l'élément d'actionnement 135. Le couvercle 150 comporte une fenêtre de visualisation 151 permettant à l'utilisateur de voir la zone d'affichage formée conjointement par lesdites parties de bord radialement externes 112, 122. Ainsi, ledit compteur peut avantageusement être pré-assemblé pour former une unité de compteur, ladite unité de compteur pouvant comporter des moyens de fixation au corps 201 du dispositif de distribution de produit fluide 200.

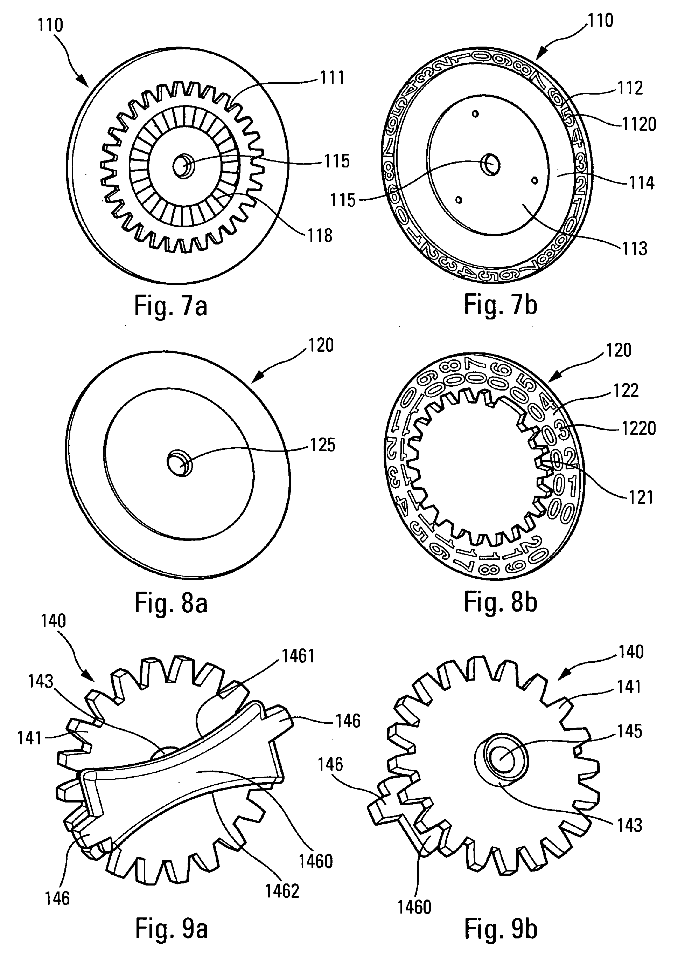

Comme représenté sur les figures 7a et 7b, ledit premier élément de comptage 11a peut comporter une première denture périphérique 118 coopérant à chaque actionnement avec au moins une, de préférence deux pattes d'actionnement flexibles 131, 132 de l'organe d'actionnement 130. Une seconde denture périphérique 111 est prévue pour coopérer à chaque actionnement avec l'élément intermédiaire 140. Ledit premier élément de comptage 110 est avantageusement sensiblement en forme de disque en étant pourvu d'une ouverture centrale traversante 115 adaptée à s'emmancher autour de son axe de rotation 161. La face supérieure dudit disque comporte une première partie de bord périphérique radialement externe 112 recevant des indices de comptage 1120, tel qu'une ou plusieurs séries de chiffres de 0 à 9. L'exemple représenté sur la figure 7b montre trois séries de chiffres de 0 à 9, répartis sur ladite périphérie. La face inférieure dudit disque comporte lesdites première et seconde dentures périphériques 118, 111, comme visible sur la figure 7a. De préférence, la première denture 118 est radialement à l'intérieur de la seconde denture 111, les dents de la première denture 118 étant orientées axialement, alors que les dents de la seconde denture sont orientées radialement vers l'extérieur. La première denture 118 et les deux pattes flexibles 131, 132 forment aussi des moyens anti-retour empêchant le premier élément de comptage 110 de tourner en sens inverse à celui qui lui est impartit par l'organe d'actionnement 130. Comme visible sur la figure 7b, la face supérieure dudit premier élément de comptage 110 peut comporter une partie centrale 113 entourant l'ouverture centrale 115, prolongée radialement à l'extérieur par une partie intermédiaire 114 axialement surélevée par rapport à ladite partie centrale 113. Ladite partie intermédiaire 114 est alors prolongée radialement à l'extérieur par ladite première partie de bord périphérique 112 axialement surélevée par rapport à ladite partie intermédiaire. Cette mise en oeuvre permet de superposer le second élément de comptage 120 sur le premier élément de comptage avec une épaisseur globale réduite.

Comme représenté sur les figures 8a et 8b, ledit second élément de comptage 120 peut comporter une troisième denture périphérique 121 adaptée à coopérer tous les dix actionnements de l'organe d'actionnement 130 avec ledit élément intermédiaire 140. Avantageusement, ledit second élément de comptage est également sensiblement en forme de disque, en étant pourvu d'un orifice central 125, qui dans l'exemple représenté est borgne, et qui est adapté à s'emmancher autour de son axe de rotation 161. La face supérieure dudit disque comporte une seconde partie de bord périphérique radialement externe 122 recevant des indices de comptage 1220, tel que les chiffres de 00 à 20, répartis sur ladite périphérie. Dans cet exemple, le compteur est donc capable de compter 200 doses. La face supérieure dudit disque comporte également ladite troisième denture périphérique 121, disposée radialement à l'intérieur de ladite seconde partie de bord périphérique externe 112, ladite troisième denture périphérique 121 étant axialement surélevée par rapport à ladite seconde partie de bord périphérique externe 122. Avantageusement, après assemblage des premier et second éléments de comptage 110, 120 autour de leur axe de rotation commun 161, ladite seconde partie de bord périphérique externe 122 dudit second élément de comptage 120 est disposée radialement à l'intérieur et sensiblement en contact de ladite première partie de bord périphérique externe 112 dudit premier élément de comptage 110, les surfaces supérieures desdites première et secondes parties de bord périphérique 112, 122 étant sensiblement alignées ou coplanaires pour former la zone d'affichage qui sera visible à travers la fenêtre de visualisation 151.

Comme visible sur les figures 9a et 9b, ledit élément intermédiaire 140 peut comporter une quatrième denture périphérique 141 coopérant à chaque actionnement de l'organe d'actionnement 130 avec la seconde denture périphérique 111 dudit premier élément de comptage 110. De cette manière, chaque actionnement du dispositif de distribution 200 est transformé par l'organe d'actionnement 130 en une rotation du premier élément de comptage 110. Avantageusement, ledit élément intermédiaire 140 comporte également au moins une projection radiale 146 coopérant tous les dix actionnements de l'organe d'actionnement 130 avec la troisième denture périphérique 121 du second élément de comptage 120. Cette projection radiale 146 peut être formée sur une partie de tige radiale 1460 dudit élément intermédiaire 140. Avantageusement, ledit élément intermédiaire 140 comporte un manchon creux axial central 143, qui dans l'exemple représenté est borgne, et qui définit un orifice central 145 adapté à s'emmancher autour de son axe de rotation 162. La quatrième denture 141 et ladite partie de tige radiale 1460 sont décalées l'une de l'autre le long dudit manchon 143 en définissant entre elles un espace qui peut recevoir la première partie de bord périphérique externe 112 du premier élément de comptage 110 et la seconde partie de bord périphérique externe 122 du second élément de comptage 120. Avantageusement, ladite partie de tige radiale 1460 comporte deux projections 146 radialement externes diamétralement opposées reliées entre elles par une zone de tige cintrée dont les bords latéraux sont de forme sensiblement en arc de cercle inversés, comme clairement visible sur les figures 4 et 9a. Ainsi, dans une orientation appropriée, représentée sur la figure 4, les premier et second éléments de comptage 110, 120 peuvent être assemblés l'un après l'autre sur leur axe de rotation commun 161, après assemblage de l'élément intermédiaire 140 sur son propre axe de rotation 162. La présente invention permet ainsi d'éviter que deux des éléments rotatifs du compteur doivent être assemblées simultanément autour des deux axes de rotation décalés, comme cela est généralement le cas avec des compteurs de ce type.

Avantageusement, ledit organe d'actionnement 130 est assemblé dans ledit corps de base 160. Le couvercle 150 peut comporter une patte flexible 158, visible sur la figure 4, coopérant avec la troisième denture 121 dudit second élément de comptage 120 pour empêcher ledit second élément de comptage 120 de tourner dans un sens ou dans l'autre lorsque l'élément intermédiaire 140 ne coopère pas avec ledit second élément de comptage 120. Bien entendu, ladite patte flexible 158 peut se déformer élastiquement pour permettre une rotation dudit second élément de comptage 120 chaque fois que ledit élément intermédiaire 140 coopère avec ledit second élément de comptage 120, c'est-à-dire tous les dix actionnements dudit organe d'actionnement 130. Avantageusement, des moyens de butée sont prévus pour former une butée au déplacement axial des pattes flexibles 131, 132. Ces moyens de butée peuvent avantageusement être formés par la première projection 161 du corps de base 160 qui peut coopérer avec une fenêtre 136 de l'organe d'actionnement 130. D'autres moyens de butée sont aussi envisageables. Les pattes 131 et 132 supportent chacune un ergot respectif 1310 et 1320 qui coopère avec la première denture 118 du premier élément de comptage 110. Les formes des ergots 1310 et 1320 sont inversées de telle sorte que le premier ergot 1310 pousse une dent de la denture 111 lors de la descente du réservoir dans le corps 201 et que le second ergot 1320 tire une dent lors de la remontée du réservoir dans le corps 201. Avantageusement, les pattes flexibles 131 et 132 sont sensiblement rigides axialement et sont flexibles dans une direction perpendiculaire au déplacement axial de l'organe d'actionnement 130. Ceci permet à la patte élastique qui n'agit pas pour faire tourner le premier élément de comptage 110 de se déformer pour glisser sur la denture et s'engrener dans la prochaine dent. Les deux pattes élastiques 131 et 132 forment aussi les moyens anti-retour pour le premier élément de comptage 110. Avantageusement, l'organe d'actionnement 130 comporte des moyens élastiques 169, tels que deux lames élastiques qui coopèrent avec deux épaulements appropriés 168 du corps de base 160 pour former ressort de rappel pour l'organe d'actionnement 130. De préférence, l'organe d'actionnement 130 comporte en outre une partie axialement déformable 134 supportant l'élément d'actionnement 135. Ceci permet de poursuivre le déplacement axial de l'élément d'actionnement 135 (et donc du réservoir) après que la position de butée définie par la projection 161 et la fenêtre 136 a été atteinte. Cette butée peut être formée de telle sorte qu'exactement une rotation d'une demi-dent soit obtenue lors de la descente du réservoir (lorsque la première dent pousse la denture 111) et que la rotation de la demi-dent restante soit obtenue lors de la remontée du réservoir, et donc de l'organe d'actionnement 130 sous l'effet des moyens élastiques 169 (lorsque la seconde dent tire sur la denture 111). L'actionnement de la valve nécessitant généralement une course supérieure, et donc un déplacement axial supérieur du réservoir, la partie déformable 134 de l'organe d'actionnement 130 permet la poursuite du déplacement axial du réservoir jusqu'à son terme. Par ailleurs, ce système permet d'actionner le compteur avant le début de la distribution du produit.

La présente invention permet notamment de sensiblement simplifier le procédé d'assemblage d'un compteur. Ainsi, l'organe d'actionnement 130 est d'abord assemblé dans ledit corps de base 160, avec l'élément d'actionnement 135 s'étendant hors dudit corps de base 160 à travers ladite ouverture 165. Ensuite, l'élément intermédiaire rotatif 140 est assemblé sur sa projection 162 dudit corps de base. Après orientation appropriée dudit élément intermédiaire dans la position d'assemblage représentée sur la figure 4, le premier élément de comptage rotatif 110 puis le second élément de comptage rotatif 120 peuvent être assemblés sur leur projection 161. Enfin, le couvercle 150 peut être assemblé sur ledit corps de base 160, pour former une unité de compteur pré-assemblée.

Le compteur de l'invention présente aussi l'avantage d'être très peu épais, permettant ainsi de diminuer les dimensions externes du dispositif et de ne pas modifier substantiellement la maniabilité du dispositif. Typiquement, l'épaisseur de l'unité de compteur pré-assemblée peut être inférieure ou égale à 7 mm, avantageusement inférieure à 6 mm, éventuellement même inférieure à 5 mm.

De manière avantageuse, l'actionnement du compteur se fait en deux parties, une première partie avant distribution du produit fluide à travers l'orifice de distribution 203 du corps 201, et une seconde partie après distribution de produit fluide. Avantageusement, le compteur ne fonctionne pas pendant que le produit est effectivement distribué, et son fonctionnement sure et fiable est donc totalement indépendant de la manière dont l'utilisateur actionne le dispositif pour réaliser une distribution de produit.

Bien entendu, par rapport à la description faite ci-dessus, le compteur pourrait être réalisé d'une manière différente à celle représenté. En particulier, les formes et positions des première et seconde pattes flexibles 131 et 132 pourraient être différentes. On peut aussi envisager d'inverser les fonctions des première et seconde pattes flexibles 131, 132, à savoir que la première patte flexible 131 pourrait tirer le premier élément de comptage 110 alors que la seconde patte flexible 132 pourrait le pousser. De même, la forme de la partie déformable 134 supportant l'élément d'actionnement 135 pourrait être différente de celle représentée sur la figure 3.

D'autres modifications sont également envisageables pour l'homme du métier, sans sortir du cadre de la présente invention tel que défini par les revendications annexées.

## Revendications

1. Compteur de doses (100) pour compter le nombre de doses de produit fluide ou pulvérulent distribuées ou restant à distribuer à partir d'un dispositif de distribution de produit fluide (200), ledit compteur comportant un premier élément de comptage rotatif (110) formant la roue des unités et un second élément de comptage rotatif (120) formant la roue des dizaines, lesdits premier et second éléments de comptage (110 ;120) coopérant pour définir ensemble et afficher dans une fenêtre de visualisation (151) ledit nombre de doses, ledit premier élément de comptage (110) coopérant avec un organe d'actionnement (130) adapté à faire tourner ledit premier élément de comptage (110) à chaque actionnement dudit organe d'actionnement (130), ledit compteur comportant un élément intermédiaire rotatif (140) adapté à faire tourner ledit second élément de comptage (120) tous les dix actionnements dudit organe d'actionnement (130), lesdits premier et second éléments de comptage (110 ;120) tournant autour d'un même premier axe de rotation (161), et ledit élément intermédiaire (140) tournant autour d'un second axe de rotation (162), décalé et parallèle audit premier axe de rotation (161), **caractérisé en ce que** ledit premier élément de comptage (110) comporte une première denture périphérique (118) coopérant à chaque actionnement avec au moins une patte d'actionnement (131,132) de l'organe d'actionnement (130) et une seconde denture périphérique (111) coopérant à chaque actionnement avec ledit élément intermédiaire (140), ledit second élément de comptage (120) comportant une troisième denture périphérique (121) coopérant tous les dix actionnements de l'organe d'actionnement (130) avec ledit élément intermédiaire (140).

2. Compteur selon la revendication 1, dans lequel ladite première denture (118) est radialement à l'intérieur de la seconde denture (111), les dents de la première denture (118) étant orientées axialement et les dents de la seconde denture étant orientées radialement vers l'extérieur.

3. Compteur selon la revendication 1 ou 2, dans lequel ledit premier élément de comptage (110) est sensiblement en forme de disque pourvu d'une ouverture centrale traversante (115) adaptée à s'emmancher autour de son axe de rotation (161), la face supérieure dudit disque comportant une première partie de bord périphérique radialement externe (112) recevant des indices de comptage, tel que une ou plusieurs séries de chiffres de 0 à 9, notamment trois séries de chiffres de 0 à 9, répartis sur ladite périphérie, la face inférieure dudit disque comportant lesdites première et seconde dentures périphériques (118, 111).

4. Compteur selon la revendication 3, dans lequel la face supérieure dudit premier élément de comptage (110) comporte une partie centrale (113) entourant l'ouverture centrale (115), prolongée radialement à l'extérieur par une partie intermédiaire (114) axialement surélevée par rapport à ladite partie centrale, ladite partie intermédiaire (114) étant prolongée radialement à l'extérieur par ladite première partie de bord périphérique (112) axialement surélevée par rapport à ladite partie intermédiaire (114).

5. Compteur selon l'une quelconque des revendications précédentes, dans lequel ledit second élément de comptage (120) est sensiblement en forme de disque pourvu d'un orifice central (125), notamment borgne, adapté à s'emmancher autour de son axe de rotation (161), la face supérieure dudit disque comportant une seconde partie de bord périphérique radialement externe (122) recevant des indices de comptage (1220), tel que les chiffres de 00 à 20, répartis sur ladite périphérie, la face supérieure dudit disque comportant également ladite troisième denture périphérique (121), disposée radialement à l'intérieur de ladite seconde partie de bord périphérique externe (122), ladite troisième denture périphérique (121) étant axialement surélevée par rapport à ladite seconde partie de bord périphérique externe (122).

6. Compteur selon les revendications 3 et 5, dans lequel après assemblage des premier et second éléments de comptage (110 ;120) autour de leur axe de rotation commun (161), ladite seconde partie de bord périphérique externe (122) dudit second élément de comptage (120) est disposée radialement à l'intérieur et sensiblement en contact de ladite première partie de bord périphérique externe (112) dudit premier élément de comptage (110), les surfaces supérieures desdites première et secondes parties de bord périphérique (112 ;122) étant sensiblement alignées pour former une zone d'affichage.

7. Compteur selon l'une quelconque des revendications précédentes, dans lequel ledit élément intermédiaire (140) comporte une quatrième denture périphérique (141) coopérant à chaque actionnement de l'organe d'actionnement (130) avec une seconde denture périphérique (111) dudit premier élément de comptage (110).

8. Compteur selon l'une quelconque des revendications précédentes, dans lequel ledit élément intermédiaire (140) comporte au moins une projection radiale (146) coopérant tous les dix actionnements de l'organe d'actionnement (130) avec une troisième denture périphérique (121) du second élément de comptage (120).

9. Compteur selon la revendication 8, dans lequel ladite au moins une projection radiale (146) est formée sur une partie de tige radiale (1460) dudit élément intermédiaire (140).

10. Compteur selon la revendication 9, dans lequel ledit élément intermédiaire (140) comporte un manchon creux axial central (143), notamment borgne, définissant un orifice central (145) adapté à s'emmancher autour de son axe de rotation (162), ladite quatrième denture (141) et ladite partie de tige radiale (1460) étant décalées l'une de l'autre le long dudit manchon (143) en définissant entre elles un espace destiné à recevoir une première partie de bord périphérique externe (112) du premier élément de comptage (110) et une seconde partie de bord périphérique externe (122) du second élément de comptage (120).

11. Compteur selon la revendication 10, dans lequel ladite partie de tige radiale (1460) comporte deux projections (146) radialement externes diamétralement opposées reliées entre elles par une zone de tige cintrée dont les bords latéraux (1461 ;1462) sont de forme sensiblement en arc de cercle inversés, de sorte que dans une orientation appropriée, les premier et second éléments de comptage (110 ;120) peuvent être assemblés sur leur axe de rotation commun (161) après assemblage de l'élément intermédiaire (140) sur son propre axe de rotation (162).

12. Compteur selon l'une quelconque des revendications précédentes, dans lequel le compteur comporte un corps de base (160) et un couvercle (150), ledit corps de base (160) incorporant les deux axes de rotation (161, 162) et une ouverture (165) et ledit couvercle (150) incorporant une fenêtre de visualisation (151).

13. Compteur selon la revendication 12, dans lequel ledit compteur peut être pré-assemblé pour former une unité de compteur, ladite unité de compteur comportant des moyens de fixation à un corps (201) d'un dispositif de distribution de produit fluide (200).

14. Compteur selon la revendication 12 ou 13, dans lequel ledit organe d'actionnement (130) est assemblé dans ledit corps de base (160), ledit organe d'actionnement (130) comportant un élément d'actionnement (135) déplaçable en translation dans ladite ouverture (165) et deux pattes flexibles (131, 132) déplaçables en translation, une première patte flexible (131) coopérant avec ladite première denture (118) du premier élément de comptage (110) pour faire tourner ledit premier élément de comptage (110) dans une direction de comptage chaque fois que l'élément d'actionnement (135) est déplacé d'une position de repos vers une position d'actionnement, et une seconde patte flexible (132) coopérant avec ladite première denture (118) dudit premier élément de comptage (110) pour faire tourner ledit premier élément de comptage (110) dans la même direction de comptage lorsque l'élément d'actionnement (135) revient de sa position d'actionnement vers sa position de repos.

15. Compteur selon la revendication 14, dans lequel la première patte flexible (131) pousse une première dent respective de la première denture (118) du premier élément de comptage (110) à chaque actionnement, et la seconde patte flexible (132) tire sur une seconde dent respective de la première denture (118) dudit premier élément de comptage (110) à chaque actionnement.

16. Compteur selon la revendication 15, dans lequel les première et seconde dents de la première denture (118) sont sensiblement diamétralement opposées sur la première denture (118).

17. Compteur selon l'une quelconque des revendications 14 à 16, dans lequel l'organe d'actionnement (130) comporte une ouverture allongée (136) assemblée autour d'une projection (161) dudit corps de base (160), la coopération entre ladite projection (161) et les bords de ladite ouvertures allongée (136) lors de l'actionnement de l'organe d'actionnement (130) définissant les limites du déplacement axial desdites pattes flexibles (131, 132).

18. Compteur selon la revendication 17, dans lequel l'organe d'actionnement (130) comporte une partie élastiquement déformable (134) supportant l'élément d'actionnement (135) de sorte que ledit élément d'actionnement (135) est déplaçable en translation sur une plus grande distance que les pattes flexibles (131, 132).

19. Compteur selon l'une quelconque des revendications 12 à 18, dans lequel ledit corps de base (160) comporte deux épaulements (168) coopérant avec deux moyens élastiques (169) de l'organe d'actionnement (130), lesdits moyens élastiques (169) formant ressort de rappel pour l'organe d'actionnement (130).

20. Compteur selon l'une quelconque des revendications 12 à 19, dans lequel ledit couvercle (150) comporte une patte flexible (158) coopérant avec une troisième denture (121) dudit second élément de comptage (120) pour empêcher ledit second élément de comptage (120) de tourner pendant les actionnements de l'organe d'actionnement (130) dans lesquels l'élément intermédiaire (140) ne coopère pas avec ledit second élément de comptage (120), ladite patte flexible (158) se déformant élastiquement pour permettre une rotation dudit second élément de comptage (120) lorsque ledit élément intermédiaire coopère avec ledit second élément de comptage tous les dix actionnements dudit organe d'actionnement (130).

21. Dispositif de distribution de produit fluide ou pulvérulent (200) comportant un réservoir, un organe de distribution, tel qu'une valve doseuse, monté sur ledit réservoir, et un corps (201) incorporant un orifice de distribution (203), ledit réservoir étant déplaçable dans ledit corps (201) pour distribuer du produit fluide ou pulvérulent, **caractérisé en ce que** ledit dispositif de distribution (200) comporte un compteur (100) selon l'une quelconque des revendications précédentes.

22. Dispositif selon la revendication 21, dans lequel ledit compteur (100) est fixé latéralement au corps (201), l'actionnement dudit dispositif étant réalisé par une pression manuelle axiale de l'utilisateur sur le réservoir et l'actionnement dudit compteur étant réalisé par ledit déplacement axial dudit réservoir qui coopère avec ledit élément d'actionnement (135) de l'organe d'actionnement (130).

23. Procédé d'assemblage d'un compteur (100) selon l'une quelconque des revendications 1 à 20, **caractérisé en ce qu'**il comporte les étapes suivantes :
- fournir un corps de base (160) pourvu de deux projections parallèles (161, 162) et d'une ouverture (165),
- assembler un organe d'actionnement (130) dans ledit corps de base (160), ledit organe d'actionnement (130) comportant un élément d'actionnement (135) s'étendant hors dudit corps de base (160) à travers ladite ouverture (165) du corps de base,
- assembler un élément intermédiaire rotatif (140) sur une première (162) desdites deux projections (161, 162) dudit corps de base (160),
- assembler un premier élément de comptage rotatif (110) sur une seconde (161) desdites deux projections (161, 162) dudit corps de base (160),
- assembler un second élément de comptage rotatif (120) sur ladite seconde projection (161) dudit corps de base (160), et
- assembler un couvercle (150) sur ledit corps de base (160), pour former une unité de compteur pré-assemblée.

## Claims

1. A dose counter (100) for counting the number of doses of fluid or powder that have been dispensed or that remain to be dispensed from a fluid dispenser device (200), said counter including a first rotary counter element (110) forming a units wheel, and a second rotary counter element (120) forming a tens wheel, said first and second counter elements (110; 120) co-operating with each other to define and to display, in a viewing window (151), said number of doses, said first counter element (110) co-operating with an actuator member (130) that is adapted to cause said first counter element (110) to turn each time said actuator member (130) is actuated, said counter including an intermediate rotary element (140) that is adapted to cause said second counter element (120) to turn on every tenth actuation of said actuator member (130), said first and second counter elements (110; 120) turning about a common first pivot pin (161), and said intermediate element (140) turning about a second pivot pin (162) that is offset and parallel to said first pivot pin (161), said counter being **characterized in that** said first counter element (110) includes: a first peripheral set of teeth (118), which set, on each actuation, co-operates with at least one actuator tab (131, 132) of the actuator member (130); and a second peripheral set of teeth (111), which set, on each actuation, co-operates with said intermediate element (140), said second counter element (120) includes a third peripheral set of teeth (121), which set, on every tenth actuation of the actuator member (130), co-operates with said intermediate element (140).

2. A counter according to claim 1, wherein said first set of teeth (118) is radially inside the second set of teeth (111), the teeth of the first set of teeth (118) being oriented axially, and the teeth of the second set of teeth being oriented radially outwards.

3. A counter according to claim 1 or claim 2, wherein said first counter element (110) is substantially in the shape of a disk provided with a central through opening (115) that is adapted to be engaged around its pivot pin (161), the top face of said disk including a first radially-outer peripheral edge portion (112) that receives counter indices, such as one or more series of numbers from 0 to 9, in particular three series of numbers from 0 to 9, distributed over said periphery, and the bottom face of said disk including said first and second peripheral sets of teeth (118, 111).

4. A counter according to claim 3, wherein the top face of said first counter element (110) includes a central portion (113) that surrounds the central opening (115) and that is extended radially outwards by an intermediate portion (114) that is raised axially relative to said central portion, said intermediate portion (114) being extended radially outwards by said first peripheral edge portion (112) that is raised axially relative to said intermediate portion (114).

5. A counter according to any preceding claim, wherein said second counter element (120) is substantially in the shape of a disk provided with a central orifice (125), in particular a blind orifice, that is adapted to be engaged around its pivot pin (161), the top face of said disk including a second radially-outer peripheral edge portion (122) that receives counter indices (1220), such as numbers from 00 to 20, distributed over said periphery, the top face of said disk also including said third peripheral set of teeth (121), disposed radially inside said second outer peripheral edge portion (122), said third peripheral set of teeth (121) being raised axially relative to said second outer peripheral edge portion (122).

6. A counter according to claim 3 and claim 5, wherein after assembling the first and second counter elements (110; 120) around their common pivot pin (161), said second outer peripheral edge portion (122) of said second counter element (120) is disposed radially inside, and substantially in contact with, said first outer peripheral edge portion (112) of said first counter element (110), the top surfaces of said first and second peripheral edge portions (112; 122) being substantially in alignment, so as to form a display zone.

7. A counter according to any preceding claim, wherein said intermediate element (140) includes a fourth peripheral set of teeth (141), which set, on each actuation of the actuator member (130), co-operates with a second peripheral set of teeth (111) of said first counter element (110).

8. A counter according to any preceding claim, wherein said intermediate element (140) includes at least one radial projection (146) that, on every tenth actuation of the actuator member (130), co-operates with a third peripheral set of teeth (121) of the second counter element (120).

9. A counter according to claim 8, wherein said at least one radial projection (146) is formed on a radial rod portion (1460) of said intermediate element (140).

10. A counter according to claim 9, wherein said intermediate element (140) includes a central axial hollow sleeve (143), in particular a blind hollow sleeve, that defines a central orifice (145) that is adapted to be engaged around its pivot pin (162), said fourth set of teeth (141) and said radial rod portion (1460) being offset relative to each other along said sleeve (143), defining between them a gap for receiving a first outer peripheral edge portion (112) of the first counter element (110), and a second outer peripheral edge portion (122) of the second counter element (120).

11. A counter according to claim 10, wherein said radial rod portion (1460) comprises two diametrally-opposite radially-outer projections (146) that are interconnected by a curved rod zone having side edges (1461; 1462) that are in the shape of circular arcs facing in substantially opposite directions, such that in an appropriate orientation, the first and second counter elements (110; 120) may be assembled on their common pivot pin (161), after the intermediate element (140) has been assembled on its own pivot pin (162).

12. A counter according to any preceding claim, wherein the counter includes a base body (160) and a lid (150), said base body (160) incorporating the two pivot pins (161, 162) and an opening (165), and said lid (150) incorporating a viewing window (151).

13. A counter according to claim 12, wherein said counter may be pre-assembled so as to form a counter unit, said counter unit including fastener means for fastening to a body (201) of a fluid dispenser device (200).

14. A counter according to claim 12 or claim 13, wherein said actuator member (130) is assembled in said base body (160), said actuator member (130) including an actuator element (135) that is movable in translation in said opening (165), and two flexible tabs (131, 132) that are movable in translation, a first flexible tab (131) co-operating with said first set of teeth (118) of the first counter element (110) so as to cause said first counter element (110) to turn in a counting direction each time the actuator element (135) is moved from a rest position to an actuated position, and a second flexible tab (132) co-operating with said first set of teeth (118) of said first counter element (110) so as to cause said first counter element (110) to turn in the same counting direction when the actuator element (135) returns from its actuated position to its rest position.

15. A counter according to claim 14, wherein, on each actuation, the first flexible tab (131) pushes a respective first tooth of the first set of teeth (118) of the first counter element (110), and the second flexible tab (132) pulls on a respective second tooth of the first set of teeth (118) of said first counter element (110).

16. A counter according to claim 15, wherein the first and second teeth of the first set of teeth (118) are substantially diametrally opposite in the first set of teeth (118).

17. A counter according to any one of claims 14 to 16, wherein the actuator member (130) includes an elongate opening (136) that is engaged around a projection (161) of said base body (160), the co-operation between said projection (161) and the edges of said elongate opening (136), while the actuator member (130) is being actuated, defining the limits of the axial movement of said flexible tabs (131, 132).

18. A counter according to claim 17, wherein the actuator member (130) includes an elastically-deformable portion (134) that supports the actuator element (135), such that said actuator element (135) is movable in translation over a greater distance than the flexible tabs (131, 132).

19. A counter according to any one of claims 12 to 18, wherein said base body (160) includes two shoulders (168) that co-operate with two resilient means (169) of the actuator member (130), said resilient means (169) forming a return spring for the actuator member (130).

20. A counter according to any one of claims 12 to 19, wherein said lid (150) includes a flexible tab (158) that co-operates with a third set of teeth (121) of said second counter element (120), so as to prevent said second counter element (120) from turning while the actuator member (130) is being actuated, the intermediate element (140) not co-operating with said second counter element (120), said flexible tab (158) deforming resiliently so as to make it possible for said second counter element (120) to turn when said intermediate element co-operates with said second counter element, on every tenth actuation of said actuator member (130).

21. A fluid or powder dispenser device (200) comprising a reservoir, a dispenser member, such as a metering valve, that is mounted on said reservoir, and a body (201) incorporating a dispenser orifice (203), said reservoir being movable in said body (201) so as to dispense the fluid or powder, said dispenser device (200) being **characterized in that** it includes a counter (100) according to any preceding claim.

22. A device according to claim 21, wherein said counter (100) is fastened on a face of the body (201), said device being actuated by the user pressing axially on the reservoir, and said counter being actuated by said axial movement of said reservoir that co-operates with said actuator element (135) of the actuator member (130).

23. A method of assembling a counter (100) according to any one of claims 1 to 20, said method being **characterized in that** it comprises the following steps:
· providing a base body (160) that is provided with two parallel projections (161, 162) and with an opening (165);
· assembling an actuator member (130) in said base body (160), said actuator member (130) including an actuator element (135) that extends out from said base body (160) through said opening (165) of the base body;
· assembling an intermediate rotary element (140) on a first (162) of said two projections (161, 162) of said base body (160);
· assembling a first rotary counter element (110) on a second (161) of said two projections (161, 162) of said base body (160);
· assembling a second rotary counter element (120) on said second projection (161) of said base body (160); and
· assembling a lid (150) on said base body (160), so as to form a pre-assembled counter unit.

## Patentansprüche

1. Dosiszähler (100) zum Zählen der Anzahl an Dosen eines Fluid- oder Pulverprodukts, die aus einer Ausgabevorrichtung für Fluidprodukt (200) ausgegeben wurden oder noch auszugeben sind, wobei der Zähler ein erstes drehbares Zählelement (110), das das Einheitenrad bildet, sowie ein zweites drehbares Zählelement (120), das das Zehnerrad bildet, aufweist, wobei das erste und das zweite Zählelement (110; 120) zusammenwirken, um zusammen die Anzahl der Dosen zu bestimmen und diese in einem Sichtfenster (151) anzuzeigen, wobei das erste Zählelement (110) mit einem Betätigungsmittel (130) zusammenwirkt, das dazu geeignet ist, bei jeder Betätigung des Betätigungsmittels (130) das erste Zählelement (110) zu drehen, wobei der Zähler ein drehbares Zwischenelement (140) aufweist, das dazu geeignet ist, bei jeder zehnten Betätigung des Betätigungsmittels (130) das zweite Zählelement (120) zu drehen, wobei sich das erste und das zweite Zählelement (110; 120) um eine gleiche erste Drehachse (161) drehen und wobei sich das Zwischenelement (140) um eine zweite Drehachse (162) dreht, die zur ersten Drehachse (161) versetzt und parallel ist, **dadurch gekennzeichnet, dass** das erste Zählelement (110) eine erste Umfangsverzahnung (118), die bei jeder Betätigung mit mindestens einer Betätigungslasche (131, 132) des Betätigungsmittels (130) zusammenwirkt, und eine zweite Umfangsverzahnung (111) aufweist, die bei jeder Betätigung mit dem Zwischenelement (140) zusammenwirkt, wobei das zweite Zählelement (120) eine dritte Umfangsverzahnung (121) aufweist, die bei jeder zehnten Betätigung des Betätigungsmittels (130) mit dem Zwischenelement (140) zusammenwirkt.

2. Zähler nach Anspruch 1, wobei die erste Verzahnung (118) in Bezug auf die zweite Verzahnung (111) radial innen liegt, wobei die Zähne der ersten Verzahnung (118) axial und die Zähne der zweiten Verzahnung radial nach außen ausgerichtet sind.

3. Zähler nach Anspruch 1 oder 2, wobei das erste Zählelement (110) in etwa in Form einer Scheibe vorliegt, die mit einer durchgehenden mittleren Öffnung (115) versehen ist, die dazu geeignet ist, um ihre Drehachse (161) herum eingesetzt zu werden, wobei die Oberseite der Scheibe einen ersten radial äußeren Umfangsrandteil (112) aufweist, der Zählwerte aufnimmt, wie eine oder mehrere Reihen von Ziffern 0 bis 9, insbesondere drei Reihen von Ziffern 0 bis 9, die auf dem Umfang verteilt sind, wobei die Unterseite der Scheibe die erste und die zweite Umfangsverzahnung (118, 111) aufweist.

4. Zähler nach Anspruch 3, wobei die Oberseite des ersten Zählelements (110) einen Mittelteil (113) aufweist, der die mittlere Öffnung (115) umgibt und durch einen Zwischenteil (114) radial nach außen verlängert ist, der in Bezug auf den Mittelteil axial erhöht ist, wobei der Zwischenteil (114) durch den ersten Umfangsrandteil (112), der in Bezug auf den Zwischenteil (114) axial erhöht ist, radial nach außen verlängert ist.

5. Zähler nach einem der vorhergehenden Ansprüche, wobei das zweite Zählelement (120) in etwa in Form einer Scheibe vorliegt, die mit einem Mittelloch (125), insbesondere einem Blindloch, versehen ist, das dazu geeignet ist, um seine Drehachse (161) herum eingesetzt zu werden, wobei die Oberseite der Scheibe einen zweiten radial äußeren Umfangsrandteil (122) aufweist, der Zählwerte (1220), wie die Ziffern von 00 bis 20, aufnimmt, die auf dem Umfang verteilt sind, wobei die Oberseite der Scheibe auch die dritte Umfangsverzahnung (121) aufweist, die in Bezug auf den zweiten äußeren Umfangsrandteil (122) radial innen angeordnet ist, wobei die dritte Umfangsverzahnung (121) in Bezug auf den zweiten äußeren Umfangsrandteil (122) axial erhöht ist.

6. Zähler nach den Ansprüchen 3 und 5, wobei nach Montage des ersten und des zweiten Zählelements (110; 120) um ihre gemeinsame Drehachse (161) der zweite äußere Umfangsrandteil (122) des zweiten Zählelements (120) radial innen und in etwa in Kontakt mit dem ersten äußeren Umfangsrandteil (112) des ersten Zählelements (110) angeordnet ist, wobei die oberen Flächen des ersten und des zweiten Umfangsrandteils (112; 122) in etwa ausgerichtet sind, um einen Anzeigebereich zu bilden.

7. Zähler nach einem der vorhergehenden Ansprüche, wobei das Zwischenelement (140) eine vierte Umfangsverzahnung (141) aufweist, die bei jeder Betätigung des Betätigungsmittels (130) mit einer zweiten Umfangsverzahnung (111) des ersten Zählelements (110) zusammenwirkt.

8. Zähler nach einem der vorhergehenden Ansprüche, wobei das Zwischenelement (140) mindestens einen radialen Vorsprung (146) aufweist, der bei jeder zehnten Betätigung des Betätigungsmittels (130) mit einer dritten Umfangsverzahnung (121) des zweiten Zählelements (120) zusammenwirkt.

9. Zähler nach Anspruch 8, wobei der mindestens eine radiale Vorsprung (146) auf einem Teil einer radialen Stange (1460) des Zwischenelements (140) gebildet ist.

10. Zähler nach Anspruch 9, wobei das Zwischenelement (140) eine hohle, axiale mittlere Hülse (143), insbesondere eine Blindhülse, aufweist, die ein Mittelloch (145) definiert, das dazu geeignet ist, um seine Drehachse (162) herum eingesetzt zu werden, wobei die vierte Verzahnung (141) und der radiale Stangenteil (1460) entlang der Hülse (143) zueinander versetzt sind und dabei zwischen sich einen Raum definieren, der dazu gedacht ist, einen ersten äußeren Umfangsrandteil (112) des ersten Zählelements (110) sowie einen zweiten äußeren Umfangsrandteil (122) des zweiten Zählelements (120) aufzunehmen.

11. Zähler nach Anspruch 10, wobei der radiale Stangenteil (1460) zwei radial äußere Vorsprünge (146) aufweist, die diametral gegenüberliegend durch einen gebogenen Stangenbereich miteinander verbunden sind, dessen Seitenränder (1416; 1462) in etwa die Form eines umgekehrten Kreisbogens haben, so dass in einer geeigneten Ausrichtung das erste und das zweite Zählelement (110; 120) auf ihrer gemeinsamen Drehachse (161) montiert werden können, nachdem das Zwischenelement (140) auf seiner eigenen Drehachse (162) montiert wurde.

12. Zähler nach einem der vorhergehenden Ansprüche, wobei der Zähler einen Grundkörper (160) und einen Deckel (150) aufweist, wobei der Grundkörper (160) die beiden Drehachsen (161, 162) sowie eine Öffnung (165) und der Deckel (150) ein Sichtfenster (151) umfasst.

13. Zähler nach Anspruch 12, wobei der Zähler vormontiert werden kann, um eine Zählereinheit zu bilden, wobei die Zählereinheit Mittel zur Befestigung an einem Körper (201) einer Ausgabevorrichtung für ein Fluidprodukt (200) aufweist.

14. Zähler nach Anspruch 12 oder 13, wobei das Betätigungsmittel (130) in dem Grundkörper (160) montiert ist, wobei das Betätigungsmittel (130) ein Betätigungselement (135), das in der Öffnung (165) verschoben werden kann, und zwei verschiebbare flexible Laschen (131, 132) aufweist, wobei eine erste flexible Lasche (131) mit der ersten Verzahnung (118) des ersten Zählelements (110) zusammenwirkt, um das erste Zählelement (110) jedes Mal in eine Zählrichtung zu drehen, wenn das Betätigungselement (135) von einer Ruheposition in eine Betätigungsposition überführt wird, und eine zweite flexible Lasche (132) mit der ersten Verzahnung (118) des ersten Zählelements (110) zusammenwirkt, um das erste Zählelement (110) in die gleiche Zählrichtung zu drehen, wenn das Betätigungselement (135) von seiner Betätigungsposition in seine Ruheposition zurückkehrt.

15. Zähler nach Anspruch 14, wobei die erste flexible Lasche (131) bei jeder Betätigung gegen einen jeweiligen ersten Zahn der ersten Verzahnung (118) des ersten Zählelements (110) gedrückt wird und die zweite flexible Lasche (132) bei jeder Betätigung an einem jeweiligen zweiten Zahn der ersten Verzahnung (118) des ersten Zählelements (110) zieht.

16. Zähler nach Anspruch 15, wobei der erste und der zweite Zahn der ersten Verzahnung (118) sich auf der ersten Verzahnung (118) in etwa diametral gegenüberliegen.

17. Zähler nach einem der Ansprüche 14 bis 16, wobei das Betätigungsmittel (130) eine längliche Öffnung (136) aufweist, die um einen Vorsprung (161) des Grundkörpers (160) montiert ist, wobei das Zusammenwirken zwischen dem Vorsprung (161) und den Rändern der länglichen Öffnung (136) bei der Betätigung des Betätigungsmittels (130) die Grenzen der axialen Verschiebung der flexiblen Laschen (131, 132) definiert.

18. Zähler nach Anspruch 17, wobei das Betätigungsmittel (130) einen elastisch verformbaren Teil (134) aufweist, der das Betätigungselement (135) derart trägt, dass das Betätigungselement (135) über eine weitere Entfernung verschoben werden kann als die flexiblen Laschen (131, 132).

19. Zähler nach einem der Ansprüche 12 bis 18, wobei der Grundkörper (160) zwei Schultern (168) aufweist, die mit zwei elastischen Mitteln (169) des Betätigungsmittels (130) zusammenwirken, wobei die elastischen Mittel (169) eine Spannfeder für das Betätigungsmittel (130) bilden.

20. Zähler nach einem der Ansprüche 12 bis 19, wobei der
Deckel (150) eine flexible Lasche (158) aufweist, die mit einer dritten Verzahnung (121) des zweiten Zählelements (120) zusammenwirkt, um zu verhindern, dass sich das zweite Zählelement (120) während der Betätigungen des Betätigungsmittels (130) dreht, wobei das Zwischenelement (140) nicht mit dem zweiten Zählelement (120) zusammenwirkt, wobei sich die flexible Lasche (158) elastisch verformt, um eine Drehung des zweiten Zählelements (120) zu erlauben, wenn das Zwischenelement bei jeder zehnten Betätigung des Betätigungsmittels (130) mit dem zweiten Zählelement zusammenwirkt.

21. Ausgabevorrichtung für ein Fluid- oder Pulverprodukt (200), aufweisend ein Behältnis, eine Ausgabeeinrichtung, wie ein Dosierventil, das auf dem Behältnis montiert ist, und einen Körper (201), der eine Ausgabeöffnung (203) umfasst, wobei das Behältnis in dem Körper (201) verschiebbar ist, um ein Fluid- oder Pulverprodukt auszugeben, **dadurch gekennzeichnet, dass** die Ausgabevorrichtung (200) einen Zähler (100) nach einem der vorhergehenden Ansprüche aufweist.

22. Vorrichtung nach Anspruch 21, wobei der Zähler (100) seitlich an dem Körper (201) befestigt ist, wobei die Betätigung der Vorrichtung durch einen manuellen axialen Druck auf das Behältnis durch den Benutzer erfolgt und die Betätigung des Zählers durch die axiale Verschiebung des Behältnisses erfolgt, das mit dem Betätigungselement (135) des Betätigungsmittels (130) zusammenwirkt.

23. Montageverfahren für einen Zähler (100) nach einem der Ansprüche 1 bis 20, **gekennzeichnet durch** die folgenden Schritte:
- Bereitstellen eines Grundkörpers (160), der mit zwei parallelen Vorsprüngen (161, 162) und einer Öffnung (165) versehen ist,
- Montieren eines Betätigungsmittels (130) in dem Grundkörper (160), wobei das Betätigungsmittel (130) ein Betätigungselement (135) aufweist, das sich **durch** die Öffnung (165) des Grundkörpers außerhalb des Grundkörpers (160) erstreckt,
- Montieren eines drehbaren Zwischenelements (140) auf einem ersten (162) der beiden Vorsprünge (161, 162) des Grundkörpers (160),
- Montieren eines ersten drehbaren Zählelements (110) auf einem zweiten (161) der beiden Vorsprünge (161, 162) des Grundkörpers (160),
- Montieren eines zweiten drehbaren Zählelements (120) auf dem zweiten Vorsprung (161) des Grundkörpers (160), und
- Montieren eines Deckels (150) auf dem Grundkörper (160), um eine vormontierte Zählereinheit zu bilden.
